(19) Europäisches Patentamt
European Patent Office
Office européen des brevets

(11) Veröffentlichungsnummer: **0 147 824**
**B1**

(12) # EUROPÄISCHE PATENTSCHRIFT

(45) Veröffentlichungstag der Patentschrift: **19.04.89**

(51) Int. Cl.⁴: **C 07 F 15/00,** C 07 C 45/50

(21) Anmeldenummer: **84116071.6**

(22) Anmeldetag: **21.12.84**

(54) **Verfahren zur Abtrennung und Wiedergewinnung von Rhodium aus den Produkten der Oxosynthese.**

(30) Priorität: **29.12.83 DE 3347406**

(43) Veröffentlichungstag der Anmeldung: **10.07.85 Patentblatt 85/28**

(45) Bekanntmachung des Hinweises auf die Patenterteilung: **19.04.89 Patentblatt 89/16**

(84) Benannte Vertragsstaaten: **DE FR GB IT NL SE**

(56) Entgegenhaltungen:
**DE-A-2 627 354**
**DE-A-3 126 265**
**FR-A-2 489 308**
**US-A-4 298 499**
**US-A-4 400 547**

(73) Patentinhaber: **HOECHST AKTIENGESELLSCHAFT, Postfach 80 03 20, D-6230 Frankfurt am Main 80 (DE)**

(72) Erfinder: **Dämbkes, G., Dr., Dipl.- Chem., Nibelungenstrasse 65, D-4220 Dinslaken (DE)**
Erfinder: **Hahn, H.- D., Dr., Dipl.- Chem., Burgstrasse 21, D-4200 Oberhausen 11 (DE)**
Erfinder: **Hibbel, Josef, Dipl.- Ing., Bruchsteg 13, D-4200 Oberhausen 11 (DE)**
Erfinder: **Materne, Winfried, Dipl.- Ing., Osterfeldstrasse 26, D-5802 Wetter 4 (DE)**

**EP 0 147 824 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft ein Verfahren zur Abtrennung von Rhodium aus den Produkten der Oxosynthese, wobei Rhodium allein, d. h. ohne speziellen Komplexbildner, wie organische Phosphine, als Katalysator verwendet wurde.

Bei der unter dem Namen Oxosynthese oder Hydroformylierung bekannten Anlagerung von Kohlenmonoxid und Wasserstoff an olefinischen Doppelbindungen zur Herstellung von Aldehyden und Alkoholen werden als Katalysatoren Metalle oder Metallverbindungen eingesetzt, die unter den Reaktionsbedingungen Carbonyle oder Carbonylhydride bilden.

Der klassische und auch heute noch am häufigsten eingesetzte Katalysator ist Kobalt, daneben fand schon frühzeitig, wenn auch in begrenztem Umfang, Rhodium als Katalysator Anwendung.

Neue Verfahren verwenden Katalysatorsysteme, die Rhodium in Kombination mit einem Komplexbildner enthalten. Beispiele für solche Komplexbildner sind organische Phosphine, wie das in Wasser nicht lösliche Triphenylphosphin, oder die wasserlöslichen Triphenylphosphin-trisulfonate und Triphenylphosphintricarboxylate.

Der besondere Vorteil der Anwendung von einfachen Rhodiumkatalysatoren, d. h. solchen ohne zusätzlichen Komplexbildner, liegt in der hohen Reaktionsgeschwindigkeit, die erzielt werden kann. Sie übersteigt die mit Kobaltkatalysatoren erreichbare Reaktionsgeschwindigkeit um den Faktor $10^2$ bis $10^4$. Darüber hinaus werden mit Rhodiumkatalysatoren erheblich geringere Mengen Nebenprodukte gebildet, als mit Kobaltkatalysatoren.

Gegenüber Verfahren mit Katalysatorsystemen aus Rhodium und einem Komplexbildner zeichnen sich Verfahren, die einfache Rhodiumkatalysatoren einsetzen, durch höhere Raumzeitausbeute und höheren Olefinumsatz, sowie durch weniger komplizierte Reaktionsführung und Produktausbringung aus. Die Hydroformylierung mit Rhodium ohne zusätzlichem Komplexbildner als Katalysator kann bei deutlich höherer Temperatur durchgeführt werden. Dadurch wird eine problemlose Nutzung der beträchtlichen Reaktionswärme möglich. Für Kobaltkatalysatoren vorgesehene Reaktoren können ohne größere Änderung auch mit Rhodiumkatalysatoren betrieben werden.

Erhebliche Schwierigkeiten bereiten jedoch die annähernd verlustfreie Abtrennung und Wiedergewinnung des Rhodiums, das ohne Komplexbildner als Katalysator eingesetzt wird. Es findet sich nach Beendigung der Umsetzung als Carbonylverbindung im Hydroformylierungsprodukt gelöst. Zur Aufarbeitung wird das Oxorohprodukt mehrstufig entspannt, indem man den Druck von Synthesedruck, d. h. etwa 250 bis 300 bar, zunächst auf 15 bis 25 bar reduziert. Hierbei wird im Rohprodukt gelöstes Synthesegas frei. Anschließend kann man auf Normaldruck entspannen. Vor der Reindarstellung durch Destillation oder der Weiterverarbeitung des Reaktionsproduktes müssen die Rhodiumverbindungen entfernt werden, die im Rohprodukt in einer Konzentration von nur wenigen ppm homogen gelöst sind. Außer durch die niedrige Konzentration können Schwierigkeiten noch dadurch auftreten, daß das Rhodium bei der Entspannung teilweise in metallische Form übergeht oder daß sich mehrkernige Rhodiumcarbonyle bilden. In beiden Fällen kommt es dann zur Ausbildung eines heterogenen Systems, das aus der flüssigen organischen Phase und der festen, Rhodium oder Rhodiumverbindungen enthaltenden Phase besteht.

Nach einem bekannten Verfahren (DE-PS 1 290 535) behandelt man zur Abtrennung des Rhodiums das Hydroformylierungsprodukt bei erhöhter Temperatur mit der wässrigen Lösung einer organischen Säure, trennt die entstandene wässrige Lösung der Rhodiumverbingung von der organischen Phase ab und führt sie, gegebenenfalls nach vorheriger Aufarbeitung, wieder der Hydroformylierungsstufe zu.

Diese Arbeitsweise hat sich zwar bewährt, sie liefert aber im allgemeinen sehr verdünnte wässrige Rhodiumsalzlösungen, die sich nur mit erheblichem Aufwand wieder in den aktiven Katalysaton überführen lassen. Überdies benötigt man zur Vervollständigung der Umsetzung eine Reaktionszeit von 1 bis 2 Stunden, während der die empfindlichen Aldehyde Temperaturen bis zu 200°C ausgesetzt werden. In Gegenwart von Ameisensäure oder Essigsäure können dann Nebenreaktionen auftreten. Außerdem ist dieses Verfahren vorzugsweise auf Reaktionsprodukte anwendbar, die Wasser nicht oder kaum lösen. Aldehyde mit stärkerem Lösungsvermögen für Wasser wie Propionaldehyd und Butyraldehyd, können einen Teil der rhodiumhaltigen, wässrigen Lösung aufnehmen, wodurch Rhodiumverluste eintreten.

Es bestand daher die Aufgabe, eine Arbeitsweise zu entwickeln, die es erlaubt, in Hydroformylierungsprodukten gelöste Rhodiumverbindungen in einfacher Weise möglichst vollständig wiederzugewinnen.

Erfindungsgemäß wird diese Aufgabe gelöst durch ein Verfahren zur Abtrennung und Wiedergewinnung von Rhodium aus den Produkten der Oxosynthese, die keine Komplexbildner enthalten, mit Hilfe komplexbildender Reagenzien. Es ist dadurch gekennzeichnet, daß als komplexbildende Reagenzien Sulfonate und Carboxylate organischer Phosphine der allgemeinen Formel

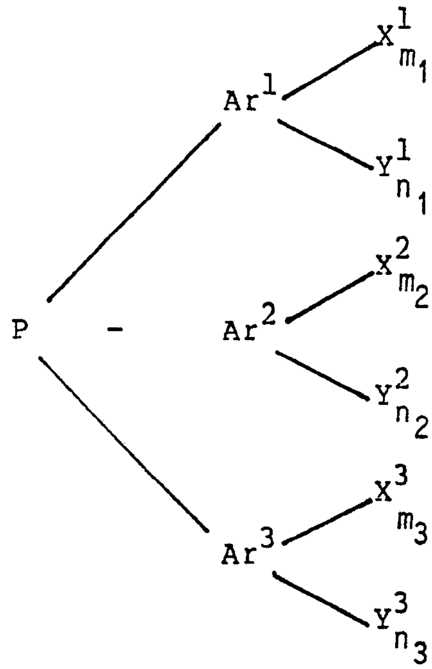

in Form einer mit dem Oxorohprodukt nicht mischbaren Lösung eingesetzt werden, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppe, in der $R^1$ und $R^2$ für geweils eine geradkettige oder verzweigte Alkylgruppe mit 1 bis 4 C-Atomen stehen, bedeuten, $X^1$, $X^2$, $X^3$ jeweils ein Sulfonat-($SO_3^-$-) und/oder ein Carboxylat-($COO^-$-)Rest ist, $m_1$, $m_2$, $m_3$ gleiche oder verschiedene ganze Zahlen von 0 bis 3 sind und mindestens eine Zahl $m_1$, $m_2$, $m_3$ gleich oder größer als 1 ist, $n_1$, $n_2$, $n_3$ gleiche oder verschiedene ganze Zahlen von 0 bis 5 sind und in den Sulfonaten und Carboxylaten Alkalimetall-, Erdalkalimetall-, Zink-, Ammonium- oder quaternäre Ammoniumionen der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht, enthalten sind.

Unter dem Begriff Oxorohprodukte wird dabei das nach Entspannen und gegebenenfalls Abkühlen anfallende Reaktionsgemisch verstanden.

Die erfindungsgemäß verwendeten Komplexbildner sind z. B. aus der FR-A-2 489 308 und der DE-A-2 627 354 bekannt. In diesen Druckschriften werden Hydroformylierungsreaktionen mit Rhodiumkomplexen dieser Verbindungen beschrieben, die in einem Zweiphasensystem ablaufen. Die Katalysatoren werden nach Beendigung der Reaktion durch Phasentrennung weidergewonnen. Eine Extraktion des Rhodiums aus der organischen in die wässrige Phase findet bei diesen Verfahren nicht statt.

Die neue Arbeitsweise erlaubt es, auf sehr einfachem Wege Rhodium schonend und unter Vermeiden von Neben- und Folgereaktionen des Hydroformylierungsproduktes aus dem Reaktionsgemisch abzutrennen.

Nach einer bevorzugten Ausführungsform des erfindungsgemäßen Verfahrens verwendet man als komplexbildende Reagenzien Verbindungen der vorstehend beschriebenen allgemeinen Formel, in der $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahl 0 oder 1 bedeuten, wobei die Summe von $m_1$, $m_2$ und $m_3$ 1, 2 oder 3 ist.

Bezogen auf Rhodium werden die Komplexbildner im Überschuß angewendet. Da man sie rezirkulieren kann, ist die Höhe des Überschusses an sich beliebig, jedoch sollen je g-Atom Rhodium mindestens 5 mol Komplexbildner vorhanden sein. Besonders bewährt hat es sich, je g-Atom Rhodium 20 bis 40 mol Komplexbildner anzuwenden.

Der Komplexbildner wird in Form einer Lösung eingesetzt. Hierbei ist darauf zu achten, daß der Komplexbildner ebenso wie der entstehende Rhodiumkomplex, im Hydroformylierungsprodukt weitgehend unlöslich, dagegen im Lösungsmittel für das komplexbildende Reagenz gut löslich ist. Selbstverständlich darf auch das Lösungsmittel mit dem Reaktionsprodukt nicht oder lediglich in ganz geringem Umfang mischbar sein.

Bei Erfüllung dieser Bedingungen bildet sich ein Zweiphasensystem aus, das aus dem Reaktionsprodukt und der Lösung des Komplexbildners bzw. der entstandenen Rhodium-Komplexverbindung besteht. Der Komplexbildner wirkt als Extraktionsmittel, d.h. im Anfangszustand befindet sich das Rhodium im Reaktionsprodukt gelöst, im Endzustand in der Lösung des Komplexbildners. Die Trennung von Reaktionsprodukt und Rhodium enthaltender Lösung erfolgt einfach durch Dekantieren der beiden Phasen voneinander.

Das bevorzugte Lösungsmittel für das komplexbildende Reagenz und den Rhodiumkomplex ist Wasser. Auch

Methanol kann als Lösungsmittel verwendet werden, sofern sichergestellt ist, daß Oxoprodukt und Methanol sich miteinander nicht mischen.

Die Konzentration des komplexbildenden Reagenzes im Lösungsmittel ist in weiten Grenzen variabel. Sie hängt insbesondere davon ab, in welchem Maße das Rhodium angereichert werden soll. Dementsprechend können nicht nur stark verdünnte, sondern sogar gesättigte Lösungen Anwendung finden. In der Regel setzt man Lösungen ein, die 0,5 bis 25 Gew.-% des Komplexbildners enthalten.

Sofern der Komplexbildner unter den Extraktionsbedingungen flüssig und im Hydroformylierungsprodukt unlöslich oder schwerlöslich und der Rhodiumkomplex im Komplexbildner löslich ist kann auf die Mitverwendung eines Lösungsmittels auch verzichtet, also der reine Komplexbildner eingesetzt werden.

Die Extraktion des Rhodiums mit dem gelösten oder reinen Komplexbildner wird bei Temperaturen von 0 bis 200°C, vorzugsweise 20 bis 100°C durchgeführt. In einzelnen Fällen hat sich aber auch eine Arbeitsweise bei einer Temperatur zwischen 120 bis 150°C bewährt.

Der neue Prozeß kann absatzweise oder kontinuierlich durchgeführt werden. Schon bei einfacher Rezirkulierung des teilweise beladenen Komplexbildners in das Reaktionsprodukt lassen sich hohe Rhodiumkonzentrationen im Extraktionsmittel erreichen. In einer anderen Variante des erfindungsgemäßen Verfahrens erfolgt die Extraktion mehrstufig, wobei das rhodiumhaltige Reaktionsprodukt im Gegenstrom zum Extraktionsmittel geführt wird.

Die Weiterbehandlung oder Weiterverwendung der das abgetrennte Rhodium enthaltenden Phase richtet sich nach den jeweiligen Gegebenheiten. So kann das Rhodium in bekannter Weise, z. B. durch Überführung in das Salz einer höheren Carbonsäure abgetrennt und wiederum als Katalysator eingesetzt werden. Es ist aber auch möglich, die aus Lösungsmittelbar als Katalysatorsystem zu verwenden.

Das erfindungsgemäße Verfahren ist zur Abtrennung und Rückgewinnung von Rhodium aus den verschiedensten Produkten der Oxosynthese geeignet. Es kann nicht nur mit Erfolg bei Rohprodukten angewandt werden, die durch Hydroformylierung olefinischer Kohlenwasserstoffe, insbesondere solchen mit 2 bis 20 Kohlenstoffatomen entstehen. Auch bei der Abtrennung von Rhodium aus Produkten der Hydroformylierung anderer olefinisch ungesättigter Verbindungen, z. B. ungesättigter Alkohole, Aldehyde, Carbonsäuren, ferner Diolefine, cyclischer Olefine wie Dicyclopentadien, hat es sich ausgezeichnet bewährt.

Die erfindungsgemäß eingesetzten Sulfonate und Carboxylate stören die anschließende Phasentrennung nicht. Schon nach wenigen Sekunden trennen sich die Phasen sehr scharf voneinander.

Nachstehend wird die Durchführung der neuen Arbeitsweise anhand einer technischen Ausführungsform beschrieben. Selbstverständlich läßt sich der erfindungsgemäße Prozeß auch in anderen Verfahrensvarianten realisieren.

Einem Reaktor 4 werden über Leitungen 1, 2 und 3 Synthesegas, Olefin und in einer organischen Phase homogen gelöster Rhodiumkatalysator zugeführt. Das über einen Standregler 5 abgezogene Produkt wird in einem Wärmetauscher 6 auf eine für die Komplexbildung geeignete Temperatur abgekühlt.

Dem aus dem Wärmetauscher 6 kommenden Produktstrom wird aus einem Trenngefäß 7 über eine Pumpe 8 in einem Rohrleitungsabschnitt 9 eine den Komplexbildner enthaltende wässrige Phase zugemischt. Auf diese Weise erreicht man eine intensive Durchmischung der Phasen und eine nahezu vollständige Extraktion des Rhodiums. Organische Phase und Wasser werden im Trenngefäß 7 getrennt. Freiwerdendes Entspannungsgas wird über eine Leitung 10, die rhodiumfreie Produktphase über eine Leitung 11 abgeführt.

Wegen der geringen Edelmetallmengen erfolgt der Abzug der mit Rhodium angereicherten wässrigen Phase über eine Leitung 12 absatzweise im Austausch gegen Ergänzungsmengen an Komplexbildnern. Dadurch wird die Kontrolle des Extraktionsprozesses wesentlich erleichtert, da nur der letzte Abschnitt der Anreicherung eine analytische Überwachung erfordert.

In den folgenden Beispielen wird die Erfindung näher beschrieben, jedoch nicht auf diese Ausführungsformen beschränkt.

Die Abkürzung TPPTS steht für Triphenylphosphin-trisulfonat. Alle Konzentrationsangaben erfolgen in Gewichtsprozent (Gew.-%).

**Biespiele**

In den Beispielen 1 bis 5 wird jeweils ein Rohprodukt der Hydroformylierung von etwa 20 bis 25°C eingesetzt, wie es nach Entspannung und Abkühlung anfällt. Zwischen der Ausschleusung des Rohproduktes aus der technischen Anlage und der Rhodiumabtrennung liegen Lagerzeiten von mehreren Stunden.

**Beispiel 1**

In einem Rührkolben werden 200 g roher Isooctylaldehyd, der

34,9 % $C_7$-Kohlenwasserstoff (überwiegend Hepten)
62,7 % Isooctylaldehyd
2,2 % Isooctylalkohol
0,2 % Höhersieder

und 3,9 ppm Rhodium enthält, mit 20 g einer 0,1 %-igen wässrigen Natrium-TPPTS-Lösung versetzt.

Das molare Verhältnis von Phosphor zu Rhodium beträgt 5. Beide Phasen werden 5 Minuten bei 50° C intensiv gerührt. Nach Beendigung des Rührens trennen sich die beiden Phasen innerhalb von 12 Sekunden ohne Emulsionsbildung. Die organische Oxorohprodukt-Phase enthält lediglich noch 1,1 ppm Rhodium, entsprechend einer Rhodiumabtrennung von 72 %.

**Beispiel 2**

Es wird wie in Beispiel 1 gearbeitet, jedoch unter Verwendung einer 0,4 %-igen Natrium-TPPTS-Lösung. Das molare Verhältnis von Phosphor zu Rhodium beträgt 20. In der organischen Phase verbleiben 1 ppm Rhodium, entsprechend einer Rhodiumabtrennung von 74 %.

**Biespiel 3**

Es wird wie in Beispiel 2 gearbeitet, jedoch bei einer Temperatur von 80° C gerührt. Die Trennung der beiden Phasen erfolgt in 9 Sekunden. Im Rohprodukt bleibt 1 ppm Rhodium zurück, entsprechend einer Rhodiumabtrennung von 74 %.

**Beispiel 4**

In einem Rundkolben mit Bodenablaß, Gaseinleitungskapillare und Rührer werden 1000 g Isooctylaldehyd der in Beispiel 1 genannten Zusammensetzung mit jeweils 100 g 20 %-iger Natrium-TPPTS-Lösung extrahiert. Über die Gaseinleitungskapillare wird zunächst Synthesegas ($CO/H_2$, 1 : 1) zur Sättigung der Mischung mit CO und Wasserstoff eingeleitet und anschließend bei 80° C intensiv gerührt. Die Rührzeit sowie die anschließende Ruhezeit betragen 30 sekunden. Darauf wird die wässrige Phase über den Bodenablaß ausgeschleust und die organische Phase erneut mit 100 g einer 20 %-igen Natrium-TPPTS-Lösung behandelt. Insgesamt wird viermal extrahiert. Die organische Phase enthält danach nur noch 0,6 ppm Rhodium entsprechend einer Rhodiumabtrennung von 85 %.

**Beispiel 5**

In der Apparatur des Beispiels 4 werden 1000 g roher Propionaldehyd, der

96,3 % Propionaldehyd
0,2 % n-Propanol
1,4 % Ethylen + Ethan
2,1 % Höhersieder

und 9,6 ppm Rhodium enthält mit jeweils 100 g 20 %-iger Natrium-TPPTS-Lösung in 5 Extraktionsschritten bei 36° C behandelt. Der Rhodiumgehalt der organischen Phase beträgt nach der 1. Extraktion 1 ppm entsprechend einer Rhodiumabtrennung von 90 % und nach der 5. Extraktion 0,6 ppm entsprechend einer Rhodiumabtrennung von 94 %.

**Beispiel 6**

In einer technischen Anlage wird bei 250 bar Diisobutylen (das etwa 90 % 2.4.4-Trimethylpenten enthält) hydroformyliert. Nach Verlassen des Reaktors wird das Produkt gekühlt und zunächst auf 20 bar, dann auf Normaldruck entspannt. In die Anlage werden stündlich 3,0 $m^3$ Diisobutylen eingespeist.

Vor der 1. Entspannungsstufe wird das Reaktionsprodukt folgender Zusammensetzung

71 % Isononylaldehyd
2 % Isononylalkohol
22 % Diisobutylen
4 % Isooctan
1 % Höhersieder

unter Synthesedruck mit 80 l/h einer 11 %-igen Natrium-TPPTS-Lösung versetzt. Das entspricht einem molaren Verhältnis von Phosphor zu Rhodium von etwa 40. Die Vermischung der Phasen erfolgt lediglich bei der Entspannung in dem kurzen Leitungsstück bis zum Niederdruckabscheider. Die Phasen trennen sich kontinuierlich im Niederdruckabscheider. Das maximale Volumen für die wässrige Phase beträgt etwa 13 Stunden. Es wird eine Temperatur von ca. 30 bis 40°C eingehalten.

Die wässrige Phase enthält 94,2 mg Rhodium je Liter Lösung und wird kontinuierlich abgezogen. Der Rhodiumgehalt der organischen Phase reduziert sich von 3,5 ppm auf Werte unter 0,2 ppm (bei einer analytischen Nachweisgrenze von 0,2 ppm), entsprechend einer Rhodiumabtrennung von 95 %.

Die Zusammensetzung der organischen Phase hat sich nicht geändert.

**Patentansprüche**

1. Verfahren zur Abtrennung und Wiedergewinnung von Rhodium aus den Produkten der Oxosynthese, die keine Komplexbildner enthalten, mit Hilfe komplexbildender Reagenzien, dadurch gekennzeichnet, daß als komplexbildende Reagenzien Sulfonate und Carboxylate organischer Phosphine der allgemeinen Formel

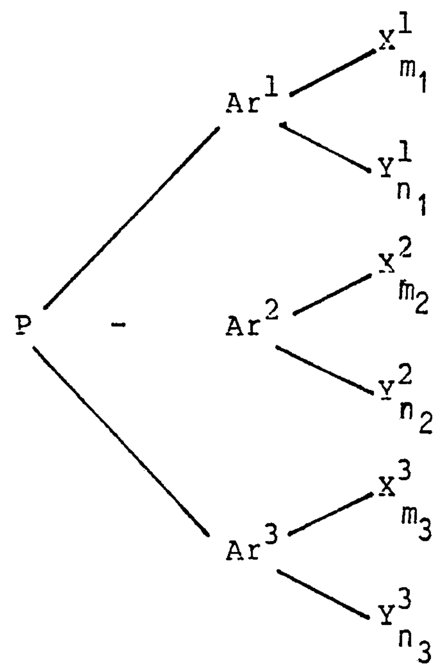

in Form einer mit dem Oxorohprodukt nicht mischbaren Lösung eingesetzt werden, wobei $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygruppe, ein Halogenatom, die OH-, CN-, $NO_2$- oder $R^1R^2N$-Gruppe, in der $R^1$ und $R^2$ für jeweils eine geradkettige oder verzweigte Alkylgruppe mit 1 bis 4 C-Atomen stehen, bedeuten, $X^1$, $X^2$, $X^3$ jeweils ein Sulfonat-($SO_3^-$-) und/oder ein Carboxylat-($COO^-$-)Rest ist, $m_1$, $m_2$, $m_3$ gleiche oder verschiedene ganze Zahlen von 0 bis 3 sind und mindestens eine Zahl $m_1$, $m_2$, $m_3$ gleich oder größer als 1 ist, $n_1$, $n_2$, $n_3$ gleiche oder verschiedene ganze Zahlen von 0 bis 5 sind und in den Sulfonaten und Carboxylaten Alkalimetall-, Erdalkalimetall-, Zink-, Ammonium- oder quaternäre Ammoniumionen der allgemeinen Formel $N(R^3R^4R^5R^6)^-$ in der $R^3$, $R^4$, $R^5$, $R^6$ jeweils für eine geradkettige oder verzweigte Alkylgruppe mit 1 bis 4 C-Atomen steht, enthalten sind.

2. Verfahren nach Anspruch 1, dadurch gekennzeichnet, daß $Ar^1$, $Ar^2$, $Ar^3$ jeweils einen Phenylrest, $X^1$, $X^2$, $X^3$ jeweils einen Sulfonatrest und $m_1$, $m_2$, $m_3$ jeweils die Zahl 0 oder 1 bedeuten, wobei die Summe von $m_1$, $m_2$ und $m_3$ 1, 2 oder 3 ist.

3. Verfahren nach Anspruch 1 oder 2, dadurch gekennzeichnet, daß je g-Atom Rhodium mindestens 5 Mol, insbesondere 20 bis 40 Mol komplexbildende Reagenz angewendet werden.

4. Verfahren nach Anspruch 1 - 3, dadurch gekennzeichnet, daß das Lösungsmittel für das komplexbildende Reagenz Wasser oder Methanol ist.

5. Verfahren nach Anspruch 4, dadurch gekennzeichnet, daß die Konzentration des komplexbildenden Reagenzes im Lösungsmittel 0,5 bis 25 Gew.-%, bezogen auf die Lösung, beträgt.

6. Verfahren nach Anspruch 1 bis 5, dadurch gekennzeichnet, daß die Abtrennung des Rhodiums bei Temperaturen von 0 bis 200° C, vorzugsweise 20 bis 100° C durchgeführt wird.

## Claims

1. A process for separating and recovering rhodium from the products of the oxo synthesis containing no complex-forming agents with the aid of complex-forming reagents characterised in that sulfonates and carboxylates of organic phosphines with the general formula

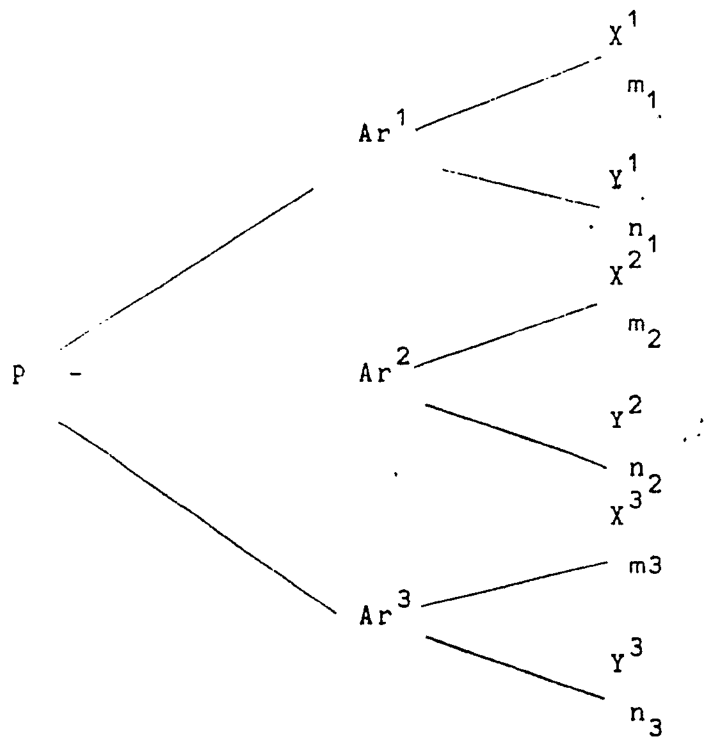

are used in the form of a solution immiscible with the oxo raw product as complex-forming reagents, where $Ar^1$, $Ar^2$ and $Ar^3$ are each a phenyl or naphthyl group, $Y^1$, $Y^2$ and $Y^3$ each denote a straight-chain or branched alkyl group with 1 to 4 carbon atoms, an alkoxy group, a halogen atom, the OH, CN, $NO_2$ or $R^1R^2N$ group where $R^1$ and $R^2$ each stand for a straight-chain or branched alkyl group with 1 to 4 cargon atoms, $X^1$, $X^2$ and $X^3$ are each a sulfonate -/$SO^{3-}$) and/or a carboxylate-($COO^-$) group, $m_1$, $m_2$ and $m_3$ are the same or different whole numbers from 0 to 3 and at least one number $m_1$, $m_2$ or $m_3$ is equal to or greater than 1, $n_1$, $n_2$ and $n_3$ are the same or different whole numbers from 0 to 5 and alkali metal, alkaline earth metal, zinc, ammonium or quaternary ammonium ions with the general formula $N(R^3R^4R^5R^6)$ where $R^3$, $R^4$, $R^5$ and $R^6$ each stand for a straight-chain or branched alkyl group with 1 to 4 carbon atoms are contained in the sulfonates and carboxylates.

2. A process according to claim 1, characterised in that $Ar^1$, $Ar^2$ and $Ar^3$ each stand for a phenyl group, $X^1$, $X^2$ and $X^3$ are each a sulfonate group and $m^1$, $m^2$ and $m^3$ each denote the number 0 or 1, the sum of $m^1$, $m^2$ and $m^3$ being 1, 2 or 3.

3. A process according to claim 1 or 2, characterised in that at least 5 moles, in particular 20 to 40 moles of complex-forming reagent are used per gramme-atom of rhodium.

4. A process according to claims 1 to 3, characterised in that the solvent for the complex-forming reagent is water or methanol.

5. A process according to claim 4, characterised in that the concentration of complex-forming reagent in the solvent is 0.5 to 25 wt. %, related to the solution.

6. A process according to claims 1 to 5, characterised in that the rhodium is separated at temperatures of 0 to 200° C, preferably 20 to 100° C.

**Revendications**

1. Procédé pour séparer et récupérer le rhodium contenu dans les produits de la synthèse Oxo exempts d'agents complexants, à l'aide de réactifs complexants, caractérisé en ce que l'on utilise en tant que réactifs complexants des sulfonates et carboxylates de phosphines organiques de formule générale

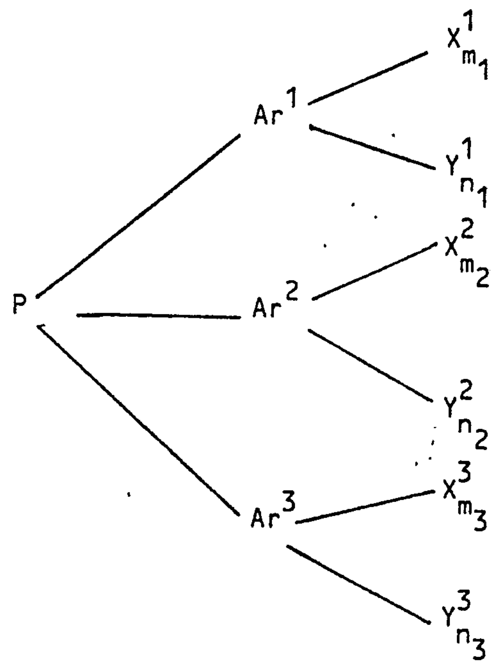

à l'état de solution non miscible avec le produit brut de la synthèse Oxo, les symboles de cette formule ayant les significations suivantes: $Ar^1$, $Ar^2$, $Ar^3$ représentent chacun un groupe phényle ou naphtyle, $Y^1$, $Y^2$, $Y^3$ représentent chacun un groupe alkyle à chaîne droite ou ramifiée en $C_1$-$C_4$, un groupe alcoxy, un atome d'halogène, un groupe OH, CN, $NO_2$ ou $R^1R^2N$ dans lequel $R^1$ et $R^2$ représentent chacun un groupe alkyle à chaîne droite ou ramifiée en $C_1$-$C_4$, $X^1$, $X^2$, $X^3$ représentent chacun un groupe sulfonate ($SO_3^-$) et/ou un groupe carboxylate ($COO^-$), $m_1$, $m_2$, $m_3$, ayant des significations identiques ou différentes, représentent des nombres entiers allant de 0 à 3 et au moins l'un des nombres $m_1$, $m_2$, $m_3$ est égal ou supérieur à 1, $n_1$, $n_2$, $n_3$ ayant des significations identiques ou différentes, représentent des nombres entiers allant de 0 à 5, et les sulfonates et carboxylates contiennent des ions de métaux alcalins, de métaux alcalino-terreux, de zinc, d'ammonium ou d'ammonium quaternaire de formule générale $N(R^3R^4R^5R^6)^+$ dans laquelle $R^3$, $R^4$, $R^5$, $R^6$ représentent chacun un groupe alkyle à chaîne droite ou ramifiée en $C_1$-$C_4$.

2. Procédé selon la revendication 1, caractérisé en ce que $Ar^1$, $Ar^2$, $Ar^3$ représentent chacun un groupe phényle, $X^1$, $X^2$, $X^3$, représentent chacun un groupe sulfonate et $m_1$, $m_2$, $m_3$ sont chacun égaux à 0 ou 1, la somme de $m_1$, $m_2$, et $m_3$ étant égale à 1, 2 ou 3.

3. Procédé selon la revendication 1 ou 2, caractérisé en ce que l'on utilise au moins 5 moles et plus spécialement de 20 à 40 moles de réactif complexant par atome-g de rhodium.

4. Procédé selon les revendications 1 à 3, caractérisé en ce que le solvant du réactif complexant est l'eau ou le méthanol.

5. Procédé selon la revendication 4, caractérisé en ce que la concentration du réactif complexant dans le solvant est de 0,5 à 25 % du poids de la solution.

6. Procédé selon les revendications 1 à 5, caractérisé en ce que la séparation du rhodium est effectuée à des températures de 0 à 200° C, de préférence de 20 à 100° C.

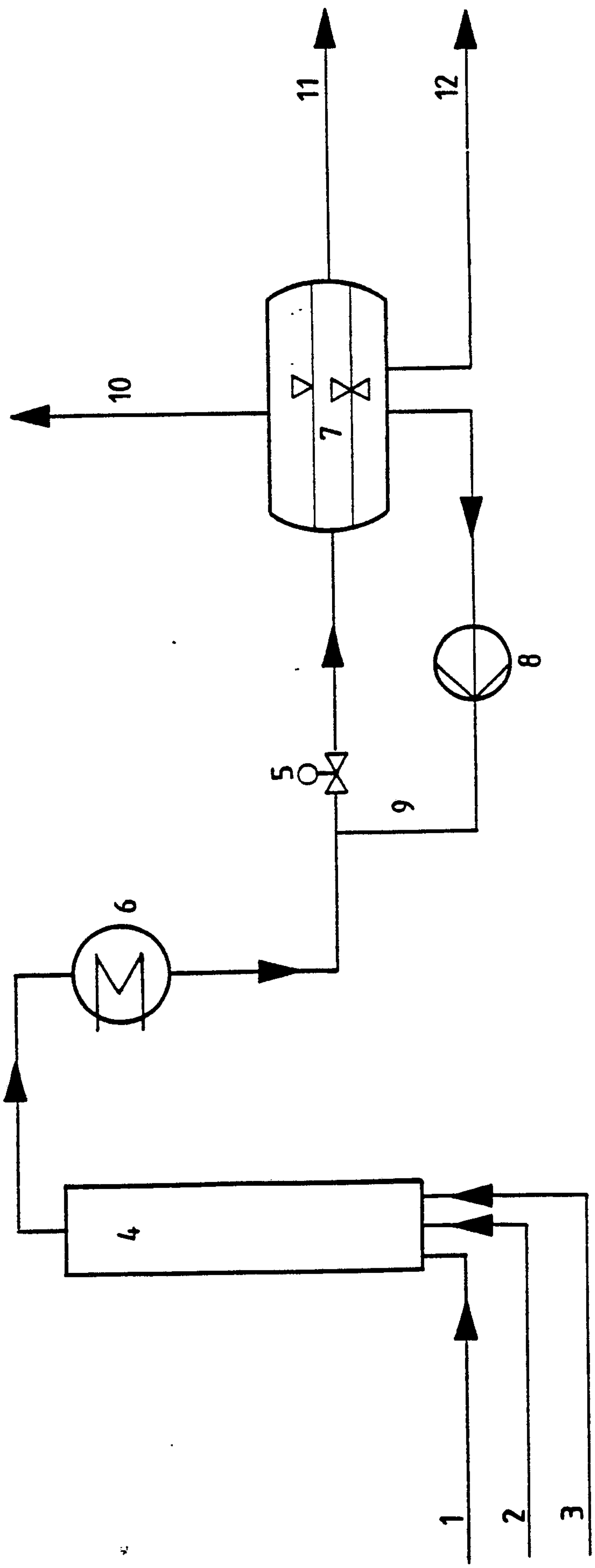
1
2
3
4
5
6
7
8
9
10
11
12